# EUROPEAN PATENT APPLICATION

(11) **EP 4 667 563 A1**
(43) Date of publication of application: **24.12.2025**
(21) Application number: 24756854.6
(22) Date of filing: 13.02.2024
(51) Int. Cl.: C12N 5/077, A61K 35/38, A61L 27/38, A61P 43/00

(54) **CELL POPULATION COMPRISING MESENCHYMAL CELLS, PHARMACEUTICAL COMPOSITION COMPRISING CELL POPULATION, EXOSOME OBTAINED FROM CELL POPULATION, AND METHOD FOR PRODUCING CELL POPULATION**

(30) Priority: 14.02.2023 JP 2023020633
(71) Applicant: Yamaguchi University, Yamaguchi 753-8511 (JP)
(72) Inventor: UENO Koji, Ube-shi, Yamaguchi 755-8505 (JP); HAMANO Kimikazu, Ube-shi, Yamaguchi 755-8505 (JP)
(74) Representative: Mewburn Ellis LLP
(86) International application number: PCT/JP2024/004737
(87) International publication number: WO 2024/172009

(57) **Abstract**

An object of the present disclosure is to provide a cell population isolated from a living organism, which contains mesenchymal cells capable of being utilized in cell sheet production and has a specific cell surface antigen molecule. A cell population isolated from a living organism and containing 80% or more of mesenchymal cells expressing at least one cell surface antigen molecule selected from the group consisting of CD13, CD59, CD49e, CD151, and CD280 is used. Preferably, the mesenchymal cells are cells collected from an intraoral tissue or a culture thereof, and the mesenchymal cells express at least three or more cell surface antigen molecules selected from the group consisting of CD13, CD59, CD49e, CD151, and CD280.

## Description

### TECHNICAL FIELD

The present disclosure relates to a cell population containing mesenchymal cells expressing a specific cell surface antigen molecule, a three-dimensional cell culture containing the cell population, a pharmaceutical composition containing the cell population, an exosome obtained from the cell population, and a method for producing the cell population.

### BACKGROUND ART

In recent years, cell transplantation therapy has attracted attention as an effective treatment for various diseases and tissue injuries. When transplanting cells, it is difficult for the cells to integrate into a tissue simply by transplanting the cells. Therefore, a cell sheet, in which cells to be transplanted are cultured in a sheet form, is used in the cell transplantation therapy. By using the cell sheet, cells to be transplanted can be fixed to a specific tissue.

The present inventors have been developing a cell sheet obtained by culturing fibroblasts, and have disclosed a method for producing a cell sheet which includes a step of culturing at a specific temperature and under low oxygen conditions for a specific period of time (see Patent Literature 1), a cell sheet containing peripheral blood mononuclear cells and fibroblasts which is used as a transplantation material for refractory skin ulcer treatment (see Patent Literature 2), and a method for producing a multilayered cell sheet which includes a step of seeding and culturing specific fibroblasts on a culture substrate supplemented with a medium (see Patent Literature 3).

Fibroblasts are used in each of the cell sheets described above. Here, fibroblasts are one of cells constituting a connective tissue, and are considered to be cells which synthesize dermal components such as collagen, elastin, and hyaluronic acid and present in the dermis. The fibroblasts are present throughout the body and have strong proliferation ability. On the other hand, characteristics of cell surface antigen molecules have not been researched so much. As a cardiac cell culture material, for example, fibroblasts expressing vascular cell adhesion molecule 1 (VCAM-1: CD106) are disclosed (see Patent Literature 4).

The fibroblasts are mesenchyme-derived cells. As for mesenchymal stem cells, mesenchymal stem cells highly expressing at least one cell surface marker selected from the group consisting of EGFR and MIC-AB (see Patent Literature 5) and mesenchymal stem cells expressing at least one cell surface marker selected from the group consisting of CD201, CD46, CD56, CD147 and CD165 (see Patent Literatures 6 and 7) have been disclosed so far.

Further, it is disclosed that as oral or gingiva-derived stem cells, a CD56-positive rate is 43% in a cell population of oral mucosa-derived fibroblasts (see Non-Patent Literature 1), a CD105-positive rate is 99% in gingiva-derived stem cells due to expression of an existing MSC cell surface marker (see Non-Patent Literature 2), gingiva-derived stem cells are positive for CD29, CD44, CD90, and CD105, and negative for CD45 and CD106 (see Non-Patent Literature 3), cell surface antigen molecules are expressed in a review on gingiva-derived mesenchymal stem cells and progenitor cells (see Non-Patent Literature 4), and a CD105-positive rate is 96% in a cell population of gingiva-derived mesenchymal stem cells (see Non-Patent Literature 5).

On the other hand, in production of a cell sheet, shortening a cell culture time greatly affects cost reduction. In general, it is said that a time until cells are doubled (a doubling time) is 25 hours to 53 hours (see Non-Patent Literature 6).

### BACKGROUND ART LITERATURE

### PATENT LITERATURE

Patent Literature 1: WO2016/043201 A1
Patent Literature 2: WO2016/068217 A1
Patent Literature 3: JP2019-38A
Patent Literature 4: JP2016-27797A
Patent Literature 5: WO2017/170925 A1
Patent Literature 6: WO2017/188403 A1
Patent Literature 7: JP2022-120128A

### NON-PATENT LITERATURE

Non-Patent Literature 1: Higa, K. et al. Future Sci. OA (2017) 3(4), FSO243
Non-Patent Literature 2: Lingqian Du. et al. Journal of Dental Sciences (2016) 11, 304e314
Non-Patent Literature 3: Ihsene Taihi et al. Stem Cell Research & Therapy (2022) 13:125
Non-Patent Literature 4: Karim M. Fawzy El-Sayed et al. Stem Cells International Volume 2016, Article ID 7154327, 16 pages.
Non-Patent Literature 5: Tomas I Mitrano et al. J Periodontol. 2010 Jun;81(6):917-25.
Non-Patent Literature 6: Endo Tomohiko et al., Journal of Dental Health (1995) 45,322-333

### SUMMARY OF INVENTION

### TECHNICAL PROBLEM

An object of the present disclosure is to provide a cell population isolated from a living organism, which contains mesenchymal cells capable of being utilized in cell sheet production and has a specific cell surface antigen molecule.

### SOLUTION TO PROBLEM

The present inventors have conducted research on the transplantation of allogeneic (non-autologous) cell sheets for the prevention of postoperative complications and the treatment of refractory skin ulcers. During the process, the inventors have focused on a gingiva excised and discarded during wisdom tooth extraction in adolescents and young adults (aged 10 to 20). Characteristics of cell surface antigen molecules in mesenchymal cells collected from an intraoral tissue were analyzed, which led to the completion of the present disclosure.

That is, the present disclosure is as follows.
[1] A cell population isolated from a living organism, the cell population containing 80% or more of mesenchymal cells expressing at least one cell surface antigen molecule selected from the group consisting of CD13, CD59, CD49e, CD151, and CD280.
[2] The cell population described in the above [1], in which the mesenchymal cells are cells collected from an intraoral tissue or a culture of the collected cells.
[3] The cell population described in the above [1] or [2], in which the mesenchymal cells express at least three or more cell surface antigen molecules selected from the group consisting of CD13, CD59, CD49e, CD151, and CD280.
[4] The cell population described in the above [1] or [2], containing 60% or more of mesenchymal cells expressing CD274.
[5] The cell population described in the above [1] or [2], containing 5% or less of mesenchymal cells expressing SSEA-3.
[6] A three-dimensional cell culture containing the cell population described in the above [1] or [2].
[7] A pharmaceutical composition containing the cell population described in the above [1] or [2].
[8] The pharmaceutical composition described in the above [7], further containing a pharmaceutically acceptable additive.
[9] The pharmaceutical composition described in the above [7] or [8], which is in a form of a three-dimensional cell culture.
[10] The pharmaceutical composition described in the above [7] or [8], which is in a form of a cell sheet.
[11] An exosome obtained from the cell population described in the above [1] or [2].
[12] A method for producing the cell population described in the above [1] or [2], the method sequentially including:
   a step (a) of collecting mesenchymal cells from a biological tissue; and
   a step (b) of culturing the collected mesenchymal cells.
[13] The method described in the above [12], further including:
   a step (c) of using an antibody which recognizes at least one cell surface antigen molecule selected from the group consisting of CD13, CD59, CD49e, CD151, and CD280 to confirm expression of the cell surface antigen molecule, after the step (b).
[14] The method described in the above [12] or [13], in which the step (b) includes culturing in a medium containing an inflammatory cytokine.
[15] The method described in the above [12] or [13], further including a step (d) of cryopreserving cultured cells, after the step (b).

Further, another aspect 1 of the present disclosure is as follows.
[1'] A cell population isolated from a living organism, the cell population containing 80% or more of mesenchymal cells expressing at least one cell surface antigen molecule selected from the group consisting of CD13, CD59, CD49e, CD151, and CD280.
[2'] The cell population described in the above [1'], in which the mesenchymal cells are cells collected from an intraoral tissue or a culture of the cells.
[3'] The cell population described in the above [1'] or [2'], in which the mesenchymal cells express at least three or more cell surface antigen molecules selected from the group consisting of CD13, CD59, CD49e, CD151, and CD280.
[4'] The cell population described in any one of the above [1'] to [3'], containing 60% or more of mesenchymal cells expressing CD274.
[5'] The cell population described in any one of the above [1'] to [4'], containing 5% or less of mesenchymal cells expressing SSEA-3.
[6'] A three-dimensional cell culture containing the cell population described in any one of the above [1'] to [5'].
[7'] A pharmaceutical composition containing the cell population described in any one of the above [1'] to [5'].
[8'] The pharmaceutical composition described in the above [7'], further containing a pharmaceutically acceptable additive.
[9'] The pharmaceutical composition described in the above [7'] or [8'], which is in a form of a three-dimensional cell culture.
[10'] The pharmaceutical composition described in any one of the above [7'] to [9'], which is in a form of a cell sheet.
[11'] An exosome obtained from the cell population described in any one of the above [1'] to [5'].
[12'] A method for producing the cell population described in any one of the above [1'] to [5'], the method sequentially including:
   a step (a) of collecting mesenchymal cells from a biological tissue; and
   a step (b) of culturing the collected mesenchymal cells.
[13'] The method described in the above [12'], further including:
   a step (c) of using an antibody which recognizes at least one cell surface antigen molecule selected from the group consisting of CD13, CD59, CD49e, CD151, and CD280 to confirm expression of the cell surface antigen molecule, after the step (b).
[14'] The method described in the above [12'] or [13'], in which the step (b) includes culturing in a medium containing an inflammatory cytokine.
[15'] The method described in any one of the above [12'] to [14'], further including a step (d) of cryopreserving cultured cells, after the step (b).

Further, another aspect 2 of the present disclosure is as follows.
[16] A method for preventing or treating an ischemic tissue, a refractory skin ulcer, a burn, an ischemic heart disease, a critical limb ischemia, or a postoperative complication, the method including:
   transplanting the pharmaceutical composition described in any one of the above [7'] to [10'] into a subject in need of transplantation.
[17] Use of the cell population described in any one of the above [1'] to [5'] for producing a pharmaceutical composition for preventing or treating an ischemic tissue, a refractory skin ulcer, a burn, an ischemic heart disease, a critical limb ischemia, or a postoperative complication.

### EFFECTS OF INVENTION

The cell population of the present disclosure enables cell transplantation therapy and production of a cell sheet for use in such therapy. When mesenchymal cells collected from a gingiva are cultured to produce a cell population, the mesenchymal cells can be easily collected from a gingiva of a healthy individual at the time of dental treatment, or the like. Further, the cell population of the present disclosure has a high cell proliferation rate, and a cell sheet can be rapidly produced.

### BRIEF DESCRIPTION OF DRAWINGS

[FIG. 1] FIG. 1 is a photograph of a cell sheet produced in Example 5.
[FIG. 2] FIG. 2 illustrates results of examining secretion of vascular endothelial growth factor (VEGF) from a cell sheet in Example 8.
[FIG. 3] FIG. 3 illustrates results of examining expression of SSEA-3 in gingival-derived mesenchymal cells in Example 9.

### DESCRIPTION OF EMBODIMENTS

### <Cell Population>

A cell population of the present disclosure is not particularly limited as long as it is a cell population isolated from a living organism, which contains 80% or more of mesenchymal cells expressing at least one cell surface antigen molecule selected from the group consisting of CD13, CD59, CD49e, CD151, and CD280, and hereinafter is also referred to as "the present cell population".

The term "mesenchymal cells," as used in the present description, refers to a collective term for cells found in mesenchymal tissue, which is a type of connective tissue formed developmentally between the ectoderm and endoderm. Examples of such cells include fibroblasts. Suitable examples of the mesenchymal cells include mesenchymal cells collected from an intraoral tissue such as gingiva or a lower part of a salivary gland (an inner cheek), an adipose tissue, bone marrow, or skin, or a culture thereof. The term "culture thereof" refers to a culture of the above-described mesenchymal cells, in which expression of a cell surface molecule in the above-described mesenchymal cells is maintained.

The term "fibroblast" in the present description refers to a cell type which contributes to formation of a connective tissue, which is a fibrous material which maintains and binds tissues and organs in a body. In normal tissue, the fibroblasts do not exhibit any particularly prominent functions. However, upon injury, the fibroblasts migrate to a damaged site, initiate production of extracellular matrix by secreting collagen, elastin, and hyaluronic acid, which help maintain a reticular structure of the tissue, and provides a function of renewing the extracellular matrix. In addition, the fibroblasts play an important role in a wound healing process, such as inducing wound contraction. Examples of a method for preparing fibroblasts include, but are not limited to, the method described in Patent Literature 2 and a method for preparing mesenchymal cells to be described later, and any cell group obtained under conditions generally used in the art for preparing a fibroblast fraction may be used.

An origin of the mesenchymal cells is not particularly limited. Examples thereof include mammals such as humans, pigs, monkeys, chimpanzees, cows, horses, sheep, goats, rabbits, dogs, cats, guinea pigs, hamsters, mice, and rats. The term "origin" is used to describe an animal species from which the cells are taken.

A cluster of differentiation (CD) classification of cell surface antigen molecules in the present description is determined based on the CD classification described in the website of HGNC (www.genenames.org/data/genegroup/#!/group/471). Gene names in the present description are determined based on gene names described in GeneCards (registered trademark) website (www.genecards.org).

The present cell population preferably contains 80% or more, more preferably 85% or more, still more preferably 90% or more, particularly preferably 95% or more, and most preferably 98% or more of the mesenchymal cells. Here, the mesenchymal cells contained in the present cell population may be one type or a combination of two or more types as long as they express at least one cell surface antigen molecule selected from the group consisting of CD13, CD59, CD49e, CD151, and CD280. That is, the present cell population may contain, for example, 80% or more, 85% or more, 90% or more, 95% or more, or 98% or more of mesenchymal cells expressing only CD13, CD59, CD49e, CD151, or CD280, and may contain 80% or more, 85% or more, 90% or more, 95% or more, or 98% or more of mesenchymal cells expressing two, three, four, or five types selected from the group consisting of CD13, CD59, CD49e, CD151, and CD280.

The mesenchymal cells in the present description are not particularly limited as long as they express at least one cell surface antigen molecule selected from the group consisting of CD13, CD59, CD49e, CD151, and CD280, and preferably express at least two selected from the group consisting of CD13, CD59, CD49e, CD151, and CD280, more preferably express at least three selected from the group consisting of CD13, CD59, CD49e, CD151, and CD280, still more preferably express at least four selected from the group consisting of CD13, CD59, CD49e, CD151, and CD280, and particularly preferably express CD13, CD59, CD49e, CD151, and CD280.

When expressing at least two selected from the group consisting of CD13, CD59, CD49e, CD151, and CD280, the at least two may be any of CD13 and CD59; CD13 and CD49e; CD13 and CD151; CD13 and CD280; CD59 and CD49e; CD59 and CD151; CD59 and CD280; CD49e and CD151; CD49e and 280; and CD151 and CD280.

When expressing at least three selected from the group consisting of CD13, CD59, CD49e, CD151, and CD280, the at least three may be any of CD13, CD59, and CD49e; CD13, CD59, and CD151; CD13, CD59, and CD280; CD13, CD49e, and CD151; CD13, CD49e, and CD280; CD13, CD151, and CD280; CD59, CD49e, and CD151; CD59, CD49e, and CD280; CD59, CD151, and CD280; and CD49e, CD151, and CD280.

When expressing at least four selected from the group consisting of CD13, CD59, CD49e, CD151, and CD280, the at least four may be any of CD13, CD59, CD49e, and CD151; CD13, CD59, CD49e, and CD280; CD13, CD59, CD151, and CD280; CD13, CD49e, CD151, and CD280; and CD59, CD49e, CD151, and CD280.

Examples of the cell population further include a cell population containing 50% or more, preferably 60% or more, more preferably 70% or more, still more preferably 80% or more, and particularly preferably 90% or more of mesenchymal cells expressing CD29, CD63, CD90, CD73, CD44, CD13, and/or HLA class I.

Examples of the cell population include a cell population containing 10% or less, preferably 5% or less, more preferably 3% or less, and still more preferably 1% or less of mesenchymal cells expressing CD304, CD106, CD34, and/or CD45.

Examples of the cell population further include a cell population containing 80% or less, preferably 70% or less, and more preferably 15 to 68% of mesenchymal cells expressing CD105.

In addition, examples of the cell population include a cell population containing 5% or less, preferably 3% or less, more preferably 1% or less, particularly preferably 0.3% or less, and most preferably 0% of mesenchymal cells expressing stage-specific embryonic antigen-3 (SSEA-3), which is known as a cell surface antigen molecule which is a marker for pluripotent stem cells.

When a pharmaceutical composition contains the cell population and is in a form of a cell sheet to be used for allogeneic transplantation, examples of the cell population include a cell population containing 50% or more, preferably 60% or more, more preferably 70% or more, still more preferably 80% or more, and particularly preferably 90% or more of mesenchymal cells expressing CD274 in order to prevent immune rejection.

Expression of the cell surface antigen molecule in a cell can be confirmed using, for example, an antibody capable of binding to the cell surface antigen molecule. Specifically, for example, in the case of confirming expression of CD13, a suspension of the cell population is prepared, and the expression of CD13 in cells constituting the cell population can be confirmed using an anti-CD13 antibody.

The term "expression" in the present description means that a protein of a cell surface antigen molecule is expressed, and it is preferable that the cell surface antigen molecule is presented on a cell surface.

In the present cell population, cells other than the mesenchymal cells are not particularly limited, and examples thereof include lymphocytic cells such as T cells, natural killer cells (NK cells), and B cells, antigen-presenting cells such as monocytes, macrophages, and dendritic cells, and granulocytes such as neutrophils, eosinophils, basophils, and mast cells.

### <Three-dimensional Cell Culture>

A three-dimensional cell culture in the present description is not particularly limited as long as it contains the present cell population. A form of the three-dimensional cell culture has a target three-dimensional structure in which cells are physically and functionally linked to each other via an adhesive molecule, an extracellular matrix, or the like. Here, the "three-dimensional cell culture" includes, but is not limited to, a cell sheet, a spheroid, an organoid, and the like. Examples of a shape of the three-dimensional cell culture include, but are not limited to, a sheet, an organ, a sphere, a tissue, a hollow, and a block. Typically, adherent cells (cells that adhere to and proliferate on a culture substrate, for example, fibroblasts), such as a cell sheet, are likely to be physically and/or functionally linked to each other to form a cell aggregate having a three-dimensional structure, and even suspended cells (that is, cells that proliferate while floating in the medium, for example, hematopoietic cells and blood cells), such as a spheroid, are physically and/or functionally linked to each other form a cell aggregate having a three-dimensional structure. In such cases, both of the adherent cells and the suspended cells fall under the scope of the three-dimensional cell culture described in the present description.

A method for producing the three-dimensional cell culture may be a method known to those skilled in the art, and is not particularly limited. Examples of the method for producing the three-dimensional cell culture include methods disclosed in literatures such as JP2012-120696A, JP2017-176025A, and JP2015-149905A.

A thickness of the three-dimensional cell culture is not particularly limited. The thickness of the three-dimensional cell culture is, for example, 0.01 mm or more and 10 mm or less, and may be 0.1 mm or more and 5 mm or less, 0.5 mm or more and 4 mm or less, or 1.0 mm or more and 3 mm or less.

In the three-dimensional culture containing the cell population, a density of cells in the three-dimensional culture to be cultured is, for example, 5 × 10¹ cells/cm³ or more and 5 × 10¹⁰ cells/cm³ or less. The density is more preferably 5 × 10² cells/cm³ or more and 5 × 10⁹ cells/cm³ or less, still more preferably 5 × 10³ cells/cm³ or more and 1 × 10⁹ cells/cm³ or less, and particularly preferably 5 × 10⁴ cells/cm³ or more and 5 × 10⁸ cells/cm³ or less.

### <Pharmaceutical Composition>

A pharmaceutical composition of the present disclosure is not particularly limited as long as it contains the cell population, and is hereinafter also referred to as "the present pharmaceutical composition". The present pharmaceutical composition may contain a pharmaceutically acceptable additive. Examples of a form of the present pharmaceutical composition include a suspension form and a three-dimensional cell culture form, and a cell sheet form is preferable.

The present pharmaceutical composition is preferably a pharmaceutical composition for ischemic tissue medical care. Here, the "ischemic tissue" refers to a tissue in a state where blood flow is reduced. In the ischemic tissue, tissue destruction occurs as a result of reduced blood flow. The present pharmaceutical composition promotes angiogenesis through secretion of VEGF in such an ischemic tissue to exhibit an effect of improving blood flow. Examples of the disease to be treated include refractory skin ulcers, such as those associated with pressure injury, arteriosclerosis obliterans, diabetes, chronic venous insufficiency (CVI), collagen disease, vasculitis, and related conditions. When a method for preventing or treating refractory skin ulcers is performed using the present pharmaceutical composition, the present pharmaceutical composition can be transplanted to an object requiring transplantation, for example, a refractory skin ulcer patient. Other examples of the disease to be treated include ischemic tissues, burns, an ischemic heart disease, critical limb ischemia, and postoperative complications (for example, anastomotic leakage in various organs, bronchial stump fistula, pancreatic fistula, and bile leakage).

Further, the present pharmaceutical composition can be used for treating refractory skin ulcers such as ischemic ulcers, collagen disease-associated ulcers, or radiation-induced ulcers and preventing postoperative complications, or as a reinforcing agent for such prevention.

Examples of the "pharmaceutically acceptable additive" in the present description include physiological saline, buffered saline, cell culture media, dextrose, water for injection, glycerol, ethanol, and combinations thereof, as well as stabilizers, solubilizers, surfactants, buffers, preservatives, tonicity agents, fillers, and lubricants.

The cell sheet form in the present pharmaceutical composition is a form having a sheet structure in which cells are physically and functionally linked to each other directly or via an adhesive molecule, an extracellular matrix, or the like. A method for producing the cell sheet may be a method known to those skilled in the art, and is not particularly limited. Examples of the method for producing the cell sheet include methods disclosed in literatures such as the above-described Patent Literatures 1 to 3, JP2011-006490A, WO2015/068505, and JP2022-8269A.

The "cell sheet" in the present description may have a single-layer structure composed of one cell layer, or may have a laminated structure composed of two or more cell layers. The laminated structure is not particularly limited, and examples thereof include a multilayer structure such as two-layer, three-layer, four-layer, and five-layer structures.

A thickness of the cell sheet in the present description is not particularly limited. The thickness of the cell sheet is, for example, 0.001 mm or more and 2.0 mm or less. The thickness of the cell sheet is more preferably 0.01 mm or more and 1.5 mm or less, still more preferably 0.03 mm or more and 1.2 mm or less, and particularly preferably 0.05 mm or more and 1.0 mm or less. When the thickness of the cell sheet is within the above range, a high cell viability in the cell sheet and excellent shape retention ability advantageous for cell transplantation can be exhibited.

The suspension form in the present description is a state in which cells are suspended in a solution such as a culture medium, PBS, or physiological saline, either alone or in contact with other cells.

### <Exosome>

An exosome in the present description is not particularly limited as long as it is an exosome obtained from the present cell population, and is hereinafter also referred to as "the present exosome". The "exosome" in the present description refers to a cell-derived vesicle composed of a lipid bilayer membrane. An exosomes is released from most cell species, can be found in many body fluids, and encapsulate nucleic acids such as DNA, mRNA, and microRNA, and proteins. In a preferred embodiment, the exosome according to the present description is an exosome obtained from the present cell population containing mesenchymal cells collected from a human gingiva. The exosome obtained from the present cell population is preferable in that it exhibits anti-inflammatory effects and promotes wound healing; that the exosome is easy to collect since mesenchymal cells can be cultured without serum; that mesenchymal cells have almost no risk of becoming cancerous, which indicates that the exosome is highly safe; and that the exosome encapsulates various growth factors. Examples of a size of the present exosome include a diameter of 20 nm to 140 nm.

The method for producing the present exosome involves culturing the present cell population, obtaining a culture supernatant, and subjecting the culture supernatant to centrifugation or ultrafiltration to remove cells and contaminants, followed by collecting the purified supernatant. The centrifugation can be performed, for example, at 1000 to 3000 rpm for 2 to 10 minutes. Thereafter, a further purified exosome may be collected using a commercially available exosome isolation kit, or an exosome may be purified and collected by ultracentrifugation (for example, 100,000 to 1,000,000 g, 30 minutes to 12 hours).

### <Method for Preparing Cell Population>

A method for preparing the cell population of the present description is not particularly limited as long as it is a method sequentially including a step (a) of collecting mesenchymal cells from a biological tissue; and a step (b) of culturing the collected mesenchymal cells, and is hereinafter also simply referred to as "the present method for preparing the cell population".

The method may include, but is not particularly limited to, a step (c) of using an antibody which recognizes at least one cell surface antigen molecule selected from the group consisting of CD13, CD59, CD49e, CD151, and CD280 to confirm expression of the cell surface antigen molecule, after the step (b). Further, the method may include, but is not limited to, a step (d) of cryopreserving cultured cells, after the step (b). A cryopreservation period and a cryopreservation method will be described later.

A method for culturing cells is not particularly limited, and any general method used in the fields of regenerative medicine, pharmaceuticals, quasi-drugs, and the like can be used. Specific examples thereof include a method in which a culture solution is added into a culture container and cells to be cultured are seeded, and the cells are cultured in an optimum environment to a target state.

A culture solution used in the method for culturing cells is a solution containing components necessary for culturing cells. The culture solution is not particularly limited as long as it is suitable for cells to be cultured. Examples of the culture solution component include saccharides, amino acids, vitamins, inorganic salts, trace metals, and additives.

These culture solution components may be blended alone or in combination of two or more. These culture solution components can be appropriately adjusted from known ones according to the cells to be cultured and the like.

Examples of the saccharides include monosaccharides such as glucose, fructose, mannose, and galactose, disaccharides such as sucrose, sucralose, trehalose, maltose, and lactose, trisaccharides such as glucosylsucrose, lactosucrose, and raffinose, tetrasaccharides such as acarbose and maltotetraose, cyclodextrins, and oligosaccharides.

Examples of the amino acids include L-glutamic acid, L-glutamine, L-arginine, glycine, L-histidine, L-isoleucine, L-leucine, L-lysine, L-methionine, L-phenylalanine, L-serine, L-threonine, L-tryptophan, L-tyrosine, L-valine, L-alanine, L-asparagine, L-aspartic acid, L-cysteine, and L-hydroxyproline.

Examples of the vitamins include sodium ascorbate, choline, folic acid, niacin, biotin, pantothenic acid, pyridoxine, riboflavin, thiamine, thymidine, and vitamin B12.

Examples of the inorganic salts include sodium chloride, sodium hydroxide, sodium sulfate, sodium phosphate, disodium hydrogen phosphate, sodium carbonate, sodium bicarbonate, potassium chloride, potassium hydroxide, potassium sulfate, potassium phosphate, dipotassium hydrogen phosphate, potassium carbonate, potassium bicarbonate, calcium chloride, calcium sulfate, calcium nitrate, calcium phosphate, calcium carbonate, magnesium chloride, magnesium sulfate, magnesium nitrate, magnesium phosphate, and magnesium carbonate.

Examples of the trace metals include iron sulfate, iron nitrate, copper sulfate, copper nitrate, and zinc sulfate.

Examples of the additives include serums such as fetal bovine serum, horse serum, and human serum, growth factors such as FGF2, EGF, HGF, VEGF, and PDGF, proteins such as albumin, antioxidants such as glutathione, ascorbic acid, and ascorbic acid derivatives, antibiotics such as penicillin and streptomycin, pH adjusters such as HEPES (2-[4-(2-hydroxyethyl)-1-piperazinyl]-ethanesulfonic acid), organic acids such as lactic acid and propionic acid, lipids such as cholesterol, fatty acids such as linoleic acid, amines such as ethanolamine and putrescine, reducing agents such as mercaptoethanol and 3-mercapto-1,2-propanediol, thickeners such as sodium alginate, polyvinylpyrrolidone, carboxymethylcellulose, and pullulan, and Rho kinase inhibitors.

When the cells to be cultured are stem cells or progenitor cells, a differentiation-inducing factor may be added to the culture solution to induce differentiation during the culture. Examples of the differentiation-inducing factor include activin A, BMP4, bFGF, VEGF, SCF, DKK1, a BMP signal inhibitor, a TGFβ/activin/NODAL signal inhibitor, a Wnt signal inhibitor, and a retinoic acid signal inhibitor.

In the step (b), the culture may be performed in a medium containing an inflammatory cytokine such as IFN-γ, interleukin-17 (IL-17), tumor necrosis factor-α (TNF-alpha (TNF-α)), IL-1, IL-2, IL-12, or IL-18.

In the step (c), the expression of the cell surface antigen molecule can be confirmed using an antibody which recognizes at least one cell surface antigen molecule selected from the group consisting of CD13, CD59, CD49e, CD151, and CD280, and the expression of each cell surface molecule may be confirmed using an antibody which recognizes CD13, CD59, CD49e, CD151, or CD280. Further, by counting the number of cells constituting the cell population and the number of cells expressing each cell surface molecule, a ratio (%) of cells expressing each cell surface molecule in the cell population can be confirmed. Whether the cells are mesenchymal cells can be confirmed using an antibody which recognizes CD90.

Specific examples of the culture solution containing the above-described culture solution components include an AIM (registered trademark) V medium, an HFDM-1 medium, an equilibration buffer such as Dulbecco's phosphate buffered saline (D-PBS) and a Hank's balanced salt solution (HBSS), a Dulbecco's Modified Eagle Medium (DMEM), a Eagle's Minimum Essential Medium (EMEM), a Minimum Essential Medium alpha Modification (α-MEM), an Iscove's Modified Dulbecco's Medium (IMDM), a Glasgow's MEM (GMEM), a Ham's F-10 medium, a Ham's F-12 medium, a Ham's F-12K medium, a RPMI medium 1640, an M-199 medium, an L-15 medium, a McCoy's 5A Medium, an MCDB105 medium, an MCDB107 medium, an MCDB131 medium, an MCDB153 medium, an MCDB201 medium, a NCTC109 medium, a NCTC135 medium, a Waymouth's MB752/1 medium, a CMRL-1066 medium, a Williams' medium E, a Brinster's BMOC-3 Medium, and an E8 medium.

Culture conditions are not particularly limited as long as cultured cells can be in an intended state. Examples of general culture conditions include an environment at 37°C and 5% CO₂ using a prepared basal culture solution. The culture conditions may be appropriately set according to the cells to be cultured. A period of cell culture is not particularly limited as long as the cultured cells are in the intended state. The period of cell culture is, for example, 28 days or less, 21 days or less, 14 days or less, 7 days or less, 5 days or less, or 3 days or less.

The number of cells seeded in the culture container is not particularly limited as long as it is suitable for the cells to be cultured and the culture container. The number of cells seeded in the culture container is, for example, 1 × 10³ cells/mL or more and 1 × 10⁷ cells/mL or less. The number of cells seeded in the culture container is more preferably 1 × 10⁴ cells/mL or more and 1 × 10⁶ cells/mL or less, still more preferably 5 × 10⁴ cells/mL or more and 5 × 10⁶ cells/mL or less, and particularly preferably 1 × 10⁵ cells/mL or more and 1 × 10⁶ cells/mL or less.

A density of the cells to be cultured is not particularly limited as long as it is suitable for the use of the cells to be cultured, the culture container, and the cultured cells. A planar density of the cells to be cultured is, for example, 1 × 10⁴ cells/cm² or more and 1 × 10⁹ cells/cm² or less. The planar density of the cells to be cultured is more preferably 1 × 10⁵ cells/cm² or more and 1 × 10⁸ cells/cm² or less, still more preferably 2 × 10⁵ cells/cm² or more and 1 × 10⁷ cells/cm² or less, and particularly preferably 5 × 10⁵ cells/cm² or more and 1 × 10⁶ cells/cm² or less.

When the pharmaceutical composition containing a cell population is a cell sheet or a three-dimensional culture containing a cell population, a density of cells in the cell sheet or the three-dimensional culture to be cultured is, for example, 5 × 10¹ cells/cm³ or more and 5 × 10¹⁰ cells/cm³ or less. The density is more preferably 5 × 10² cells/cm³ or more and 5 × 10⁹ cells/cm³ or less, still more preferably 5 × 10³ cells/cm³ or more and 1 × 10⁹ cells/cm³ or less, and particularly preferably 5 × 10⁴ cells/cm³ or more and 5 × 10⁸ cells/cm³ or less.

The mesenchymal cells in the present description may be autologous cells derived from a patient for whom the pharmaceutical composition is to be used, or may be allogenic cells derived from another target of the same type, for example, another patient.

A dose of the present pharmaceutical composition, in the case of a suspension, can be, in terms of the number of cells, 5 × 10² to 1 × 10¹² cells per administration, preferably 1 × 10⁴ cells to 1 × 10¹¹ cells per administration, and more preferably 1 × 10⁵ cells to 1 × 10¹⁰ cells per administration. It should be noted that the dosage may be administered multiple times as a single dose or may be divided into multiple doses for administration. When the present pharmaceutical composition is administered to an adult, the number of cells per body weight is generally 1 × 10 to 5 × 10¹⁰ cells/kg, preferably 1 × 10² to 5 × 10⁹ cells/kg, and more preferably 1 × 10³ to 5 × 10⁸ cells/kg. It should be noted that the dosage may be administered multiple times as a single dose or may be divided into multiple doses for administration.

### <Cryopreservation>

The present cell population, the present three-dimensional cell culture, the present pharmaceutical composition, and the present exosomes may be cryopreserved. Specifically, cryopreservation can be performed at -20°C or lower, preferably at -80°C for 1 day or more, 1 week or more and 10 years or less, 1 month or more and 5 years or less, or 1 year or more and 3 years or less. Examples of the cryopreservation methods include cryopreservation using a cell cryopreservation storage solution commonly used in biotechnology, the method described in JP2022-8269A, slow freezing, and vitrification methods. The cryopreservation period and cryopreservation method can also be applied to the step (d) of cryopreserving cultured cells after the step (b) in the method for preparing the cell population.

The cryopreserved present cell population can also be banked in a cell bank. As an example of collecting mesenchymal cells from gingival tissue, specifically, mesenchymal cells are collected from gingival tissue excised during suturing after wisdom tooth extraction from a patient who has provided consent, and are then cultured (P0). Next, the collected mesenchymal cells (P0) are seeded, and one hour later, the medium is replaced and adhesive cells are cultured. Cells (P1) obtained by separating the cultured cells from the medium can be cryopreserved and stored in a cell bank.

When utilized in regenerative medicine, for example, the banked cryopreserved cells (P1) are seeded in a plate, cultured up to P4, and then cryopreserved. When used, the cryopreserved cells can be thawed and used for transplantation. The numbers in P0, P1, and P4 mean subculture numbers.

### Examples

Hereinafter, the present disclosure will be more specifically described with reference to Examples, but the technical scope of the present disclosure is not limited thereto.

However, the present invention is not limited thereto.

### [Example 1] Preparation of Mesenchymal Cells Collected from Gingival Tissue

First, gingiva-derived cells were cultured by the following method to prepare P1 cells, P2 cells, or P3 cells, which were used in the following Examples. The numbers in P1, P2, and P3 mean subculture numbers.

### <Preparation of P1 Cells>

- Gingival tissues from five donors (No.1 to No.5) were minced with tweezers and a scalpel, and the minced gingival tissues were attached to a 6-well plate (#MS-80060S; Sumitomo Bakelite Co., Ltd.).
- One 50 mL tube was charged with 1.5 mL of bovine-derived cell culture serum NeoSERA (registered trademark) (Japan Biomedical Co., Ltd.), 15 mL of AIM-V Medium (Thermo Fisher Scientific K.K.), and 15 mL of HFDM-1(+) (Cell Science & Technology Institute, Inc.), and the culture solution was adjusted by pipetting.
- Each well of the 6-well plate was charged with 5 mL of the culture solution, and the minced gingival tissues were cultured. P0 cells growing out of the minced gingival tissues were collected, and the cells were seeded in a 90 mm Petri dish (#MS-13900S: Sumitomo Bakelite Co., Ltd.). One hour later, the medium was replaced and adhesive cells were cultured.
- A medium for one 90 mm Petri dish was AIM-V Medium 4.5 mL, HFDM-1(+) 4.5 mL, and NeoSERA 1 mL.
- P1 cells were collected when a density of the cells cultured in the 90 mm Petri dish reached 70% to 95%, the P1 cells were adjusted to 1 × 10⁶ cells/mL with STEM-CELLBANKER (registered trademark) GMP grade (product code #CB045: ZENOAQ Resource Co., LTD.), aliquoted into cryotubes at 1 mL per tube to prepare a cell preservation solution containing the P1 cells, which were cryopreserved at -80°C until use.

### <Expression of CD90 in P1 Cells>

Expression of CD90 in the P1 cells was confirmed by the following method.
- The P1 cells were adjusted to a concentration of 1 × 10⁶ cells/mL in PBS, and a 15 mL tube was placed on ice.
- A 1.5 mL tube was charged with 20 µL of anti-CD90 antibody or isotype control antibody shown in Table 1, then 100 µL of the P1 cells (1 × 10⁶ cells/mL) were added and pipetted. The tube was kept on ice, protected from light, and left to stand for 30 minutes. In Table 1, ISO means isotype control.
- A 1.5 mL tube was charged with 400 µL of PBS and pipetted, then passed through a 5 mL polystyrene round tube with a cell strainer cap (#REF352235, Becton, Dickinson and Company).
- The solution was transferred to a standard PP test tube (round bottom) (#A26428, Beckman Coulter, Inc.) and placed on ice until a sample measurement was started.
- For No.1, a fluorescence wavelength was measured with Novocyte (ACEA Biosciences Inc.), and for No.2 to No.5, a fluorescence wavelength was measured with Cytomics FC 500 (Beckman Coulter, Inc.).

**[Table 1]**

| | Manufacturer | Product code | Clone | Isotype | Product name |
|---|---|---|---|---|---|
| CD90 | Thermo Fisher Scientific (eBioscience) | 11-0909-42 | eBio5E10 (5E10) | Mouse/IgG1, kappa | CD90 (Thy-1) Monoclonal Antibody (eBio5E10 (5E10)), FITC, eBioscience^{™} |
| ISO (FITC) | BD Bioscience | 556649 | MOPC-21 | Mouse IgG1, κ | FITC Mouse IgG1, κ Isotype Control |

Results are shown in Table 2. From the results of Table 2, the obtained cell population contained 95% or more of CD90, which was a marker for mesenchymal cells, and most of the obtained P1 cell population was determined to be mesenchymal cells. When the start of culture of the minced gingival tissues was defined as day 0, the numbers of living P1 cells on day 13 of culture were 13.8 × 10⁶ cells/mL, 43.1 × 10⁶ cells/mL, 45.6 × 10⁶ cells/mL, 37 × 10⁶ cells/mL, and 22 × 10⁶ cells/mL, respectively, in order of No.1 to No.5.

**[Table 2]**

| | No.1 | No.2 | No.3 | No.4 | No.5 |
|---|---|---|---|---|---|
| CD90 | 95.1 | 99.8 | 99.9 | 99.8 | 99.8 |

### <Preparation of P2 Cells>

P2 cells were prepared by the following steps.
- The cell preservation solution containing the P1 cells and aliquoted into the cryotubes was thawed.
- A 15 mL tube was charged with 3.5 mL of AIM-V Medium (Thermo Fisher Scientific K.K.), 0.5 mL of bovine-derived cell culture serum NeoSERA (registered trademark) (Japan Biomedical Co., Ltd.), and 1 mL of the cell preservation solution containing the thawed P1 cells, followed by centrifuging at 1200 rpm for 1 minute at room temperature.
- The supernatant was removed by suction, and the P1 cells were seeded in two 10 cm dishes (#93100: BM Equipment Co., LTd.) and cultured in an incubator (37°C, 5% CO₂) for 4 days.

As a medium for the 10 cm dishes, AIM-V Medium 5 mL + HFDM-1(+) (Cell Science & Technology Institute, Inc.) + NeoSERA 1 mL was used.
- The culture solution was removed by suction from the 10 cm dishes, and 5 mL of PBS (Cell Science & Technology Institute, Inc.) was placed in one 10 cm dish to wash the inside of the dish, and then the PBS was removed by suction.
- The one 10 cm dish was charged with 2 mL of r-TE (Cell Science & Technology Institute, Inc.), which is a recombinant trypsin/EDTA solution, and the 10 cm dish was left to stand in an incubator (37°C, 5% CO₂) for 3 minutes.
- A 40 µm cell strainer (#352340: Corning Incorporated) was attached to a 50 mL tube.
- The cells were detached from the 10 cm dish by pipetting and transferred to the 50 mL tube.
- The one 10 cm dish was charged with 2 mL of s-TI (Cell Science & Technology Institute, Inc.), which is a synthetic trypsin neutralizer solution, the inside of the 10 cm dish was washed, and the washing solution was transferred to the 50 mL tube.
- The 50 mL tube was centrifuged at 1200 rpm for 1 minute at room temperature.
- After centrifugation, the supernatant was removed by suction, and the cells were suspended in 2 mL of AIM-V Medium to obtain a suspension containing P2 cells. After 10 µL of the cell suspension liquid and 10 µL of trypan blue were mixed, 10 µL of the mixed liquid was applied to a hemocytometer to count the number of cells.
- For a doubling experiment to be described later, 2.5 × 10⁵ P2 cells were seeded in each of three cell culture flasks (#MS-23800 225 cm²: Sumitomo Bakelite Co., Ltd.). As a culture solution in one cell culture flask, AIM-V Medium 25 mL + HFDM-1(+) 25 mL + NeoSERA 2 mL was used.
- With respect to remaining cells, P2 cells were adjusted to 1 × 10⁶ cells/mL with STEM-CELLBANKER (registered trademark) GMP grade (product code #CB045: ZENOAQ Resource Co., LTD.), aliquoted into cryotubes at 1 mL per tube to prepare a cell preservation solution containing the P2 cells, which were cryopreserved at -80°C until use.

### <Preparation of P3 Cells>

- In No.1, P2 cells were subcultured 3 days after seeding in a cell culture flask, and in No.2 to No.5, P2 cells were subcultured 5 days after seeding in a cell culture flask.
- In No.2 to No.5, a half amount of the medium was replaced 3 days after cell seeding.
- The culture solution was removed by suction from one cell culture flask, 25 mL of PBS was placed in one cell culture flask, the inside of the cell culture flask was washed, and then PBS was removed by suction.
- One cell culture flask was charged with 5 mL of a recombinant trypsin/EDTA solution r-TE (Cell Science & Technology Institute, Inc.), and the cell culture flask was allowed to stand in an incubator (37°C, 5% CO₂) for 3 minutes.
- A 40 µm cell strainer (#352340: Corning Incorporated) was attached to a 50 mL tube.
- The cells were detached from the cell culture flask by pipetting and transferred to the 50 mL tube.
- The one cell culture flask was charged with 5 mL of s-TI (Cell Science & Technology Institute, Inc.) as a synthetic trypsin neutralizer solution, the inside of the cell culture flask was washed, and the washing solution was transferred to the 50 mL tube.
- The 50 mL tube was centrifuged at 1200 rpm for 2 minutes at room temperature.
- After centrifugation, the supernatant was removed by suction, and the cells were suspended in 20 mL of AIM-V medium to obtain a suspension containing P3 cells. After 10 µL of the cell suspension liquid and 10 µL of trypan blue were mixed, 10 µL of the mixed liquid was applied to a hemocytometer to count the number of cells.
- For the doubling experiment, 2.5 × 10⁵ P3 cells were seeded in each of three cell culture flasks (#MS-23800 225 cm²: Sumitomo Bakelite Co., Ltd.).
- P3 cells were adjusted to 1 × 10⁶ cells/mL with STEM-CELLBANKER (registered trademark) GMP grade (product code #CB045: ZENOAQ Resource Co., LTD.), aliquoted into cryotubes at 1 mL per tube to prepare a cell preservation solution containing the P3 cells, which were cryopreserved at -80°C until use.

### [Example 2] Expression of Cell Surface Antigen Molecule of Mesenchymal Cells Collected from Gingival Tissue

Among many cell surface antigen molecules, the present inventors focused on CD13, CD29, CD59, CD49e, CD151, CD280, CD304, CD274, CD63, CD106, HLA class I, and HLA class II based on the knowledge and know-how obtained thus far, and used antibodies to examine cell surface molecules known as a marker for mesenchymal stem cells and expression of the cell surface antigen molecules.
- One cryotube (the cell preservation solution containing P3 cells) prepared in Example 1 was taken out from -80°C and thawed.
- A 15 mL tube was charged with 3.5 mL of AIM-V Medium (Thermo Fisher Scientific K.K.), 0.5 mL of bovine-derived cell culture serum NeoSERA (registered trademark) (Japan Biomedical Co., Ltd.), and 1 mL of cell preservation solution containing thawed P3 cells, followed by centrifuging at 1200 rpm for 1 minute at room temperature.
- The supernatant was removed by suction, and P3 cells were seeded in two 10 cm dishes. As a medium for one 10 cm dish, AIM-V Medium 5 mL + HFDM-1(+) (Cell Science & Technology Institute, Inc.) + NeoSERA 1 mL was used.
- The 10 cm dish was cultured in an incubator (37°C, 5% CO₂) for 4 days.
- The culture solution was removed by suction from the 10 cm dish, and 5 mL of PBS (Cell Science & Technology Institute, Inc.) was placed in one 10 cm dish to wash the inside of the dish, and then the PBS was removed by suction.
- The one 10 cm dish was charged with 2 mL of r-TE (Cell Science & Technology Institute, Inc.), which is a recombinant trypsin/EDTA solution, and the 10 cm dish was left to stand in an incubator (37°C, 5% CO₂) for 3 minutes.
- A 40 µm cell strainer (#352340: Corning Incorporated) was attached to a 50 mL tube.
- The cells were detached from the 10 cm dish by pipetting and transferred to the 50 mL tube.
- The one 10 cm dish was charged with 2 mL of s-TI (Cell Science & Technology Institute, Inc.), which is a synthetic trypsin neutralizer solution, the inside of the 10 cm dish was washed, and the washing solution was transferred to the 50 mL tube.
- The 50 mL tube was centrifuged at 1200 rpm for 1 minute at room temperature.
- After centrifugation, the supernatant was removed by suction, the cells were suspended in 2 mL of PBS to prepare a suspension liquid containing P4 cells, 10 µL of the cell suspension liquid and 10 µL of trypan blue were mixed, 10 µL of the mixture was applied to a hemocytometer to count the number of cells.
- After adjusting a cell concentration to 1 × 10⁶ cells/mL with PBS, the 50 mL tube was placed on ice.
- A 1.5 mL tube was charged with 20 µL each of antibodies shown in Table 3, then 100 µL of the cell suspension liquid (1 × 10⁶ cells/mL) was added and pipetted. The tube was kept on ice, protected from light, and left to stand for 30 minutes.
- A 1.5 mL tube was charged with 400 µL of PBS and pipetted, then passed through a 5 mL polystyrene round tube with a cell strainer cap (#REF352235: Becton, Dickinson and Company).
- The solution was transferred to a standard PP test tube (round bottom) (#A26428: Beckman Coulter, Inc.) and placed on ice until a sample measurement was started.
- A fluorescence wavelength was measured with Cytomics FC 500 (Beckman Coulter, Inc.).

**[Table 3]**

| | Manufacturer | Product code | Clone | Isotype | Product name |
|---|---|---|---|---|---|
| CD90 | Thermo Fisher Scientific (eBioscience) | 11-0909-42 | eBio5E10 (5E10) | Mouse/IgG1, kappa | CD90 (Thy 1) Monoclonal Antibody (eBio5E10 (5E10)), FITC, eBioscience^{™} |
| CD73 | BD Bioscience | 561254 | AD2 | Mouse IgG1, κ | FITC Mouse Anti-Human CD73 |
| CD44 | BD Bioscience | 555478 | G44-26 | Mouse IgG2b, κ | FITC Mouse Anti-Human CD44 |
| CD105 | BD Bioscience | 561443 | 266 | Mouse BALB/c IgG1, κ | FITC Mouse anti-Human CD105 |
| CD166 | BD Bioscience | 559263 | 3A6 | Mouse IgG1, κ | PE Mouse Anti-Human CD166 |
| CD34 | BD Bioscience | 555821 | 581 | Mouse IgG1, κ | FITC Mouse Anti-Human CD34 |
| CD45 | BD Bioscience | 555482 | HI30 | Mouse IgG1, κ | FITC Mouse Anti-Human CD45 |
| CD56 | Sigma-Aldrich | F0301-100TS | C5.9 | Mouse IgG2b κ | Monoclonal Anti-CD56-FITC, antibody produced in mouse |
| CD13 | Thermo Fisher Scientific (eBioscience) | 11-0138-42 | WM-15 (WM15) | Mouse/IgG1, kappa | CD13 Monoclonal Antibody (WM-15 (WM15)), FITC, eBioscience^{™} |
| CD29 | Thermo Fisher Scientific (eBioscience) | 11-0299-42 | TS2/16 | Mouse/IgG1, kappa | CD29 (Integrin beta 1) Monoclonal Antibody (TS2/16), FITC, eBioscience^{™} |
| CD59 | Thermo Fisher Scientific (eBioscience) | 11-0596-42 | OV9A2 | Mouse/IgG1, kappa | CD59 (Protectin) Monoclonal Antibody (OV9A2), FITC, eBioscience^{™} |
| CD49e | BD Bioscience | 555617 | IIA1 | Mouse RBF/DnJ IgG1, κ | PE Mouse Anti-Human Integrin a 5 chain (CD49e) |
| CD151 | BD Bioscience | 556057 | 14A2,H1 | Mouse BALB/c IgG1, κ | PE Mouse Anti-Human CD151 |
| CD280 | BD Bioscience | 566817 | E1/183 | Mouse IgG1, κ | PE Mouse Anti-Human CD280 |
| CD304 | BD Bioscience | 565952 | U21-1283 | Mouse IgG2b, κ | PE Mouse Anti-Human Neuropilin-1 (CD304) |
| ISO (FITC) | BD Bioscience | 556649 | MOPC-21 | Mouse IgG1, κ | FITC Mouse IgG1, κ Isotype Control |
| ISO (PE) | BD Bioscience | 555574 | G155-178 | Mouse BALB/c IgG2a, κ | PE Mouse IgG2a, κ Isotype Control |
| CD304 | BioLegends | 354503 | 12C2 | Mouse IgG2a, κ | PE anti-human CD304 (Neuropilin-1) Antibody |
| CD63 | Thermo Fisher Scientific (eBioscience) | 12-0639-42 | H5C6 | Mouse/IgG1, kappa | CD63 Monoclonal Antibody (H5C6), PE, eBioscience^{™} |
| HLA class I | BioLegend | 311404 | W6/32 | Mouse IgG2a, κ | FITC anti-human HLA-A,B,C Antibody |
| HLA class II | BD Biosciences | 550853 | Tu39 (also know | Mouse IgG2a, κ | FITC Mouse Anti-Human HLA-DR,DP,DQ |
| CD274 | Thermo Fisher Scientific (eBioscience) | 12-5983-42 | MIH1 | Mouse/IgG1, kappa | CD274 (PD-L1, B7-H1) Monoclonal Antibody (MIH1), PE, eBioscience^{™} |
| CD106 | BD Biosciences | 561679 | 51-10C9 | Mouse IgG1, κ | PE Mouse Anti-Human CD106 |

Results are shown in Table 4. As is clear from Table 4, it was confirmed that the cell populations obtained by culturing mesenchymal cells derived from any of gingival tissues of No.1 to No.5 contained 95% or more of cells expressing CD13, CD29, CD59, CD49e, CD151, or CD280. In the cell populations obtained by culturing mesenchymal cells derived from any of gingival tissues of No.1 to No.5, 97% or more of cells expressed CD90 or CD73, which are markers for mesenchymal stem cells. Further, the cell populations contained 60% or more of cells expressing CD274 and 16% to 66% of cells expressing CD105. On the other hand, in any of the above cell populations, the expression of CD34, CD45, CD56, CD304, and CD106 was not confirmed, and cells expressing HLA class II accounted for only 0% to 0.4%. The expression of CD13, CD59, CD49e, CD151, and CD280 has not been confirmed in gingival-derived cell populations until now, and thus new cell surface molecules in gingival-derived mesenchymal cells have been confirmed.

**[Table 4]**

| | No.1 | No.2 | No.3 | No.4 | No.5 |
|---|---|---|---|---|---|
| CD90 | 99.8 | 99.8 | 99.8 | 99.9 | 99.7 |
| CD73 | 98.6 | 99.3 | 97.3 | 99.3 | 99.7 |
| CD44 | 99.6 | 99.6 | 99.7 | 99.8 | 99.9 |
| CD105 | 16.4 | 65.7 | 55.2 | 66.7 | 61.3 |
| CD166 | 62.8 | 87.3 | 91.1 | 94.4 | 76.8 |
| CD34 | 0 | 0 | 0 | 0 | 0 |
| CD45 | 0 | 0 | 0 | 0 | 0 |
| CD56 | 0 | 0 | 0 | 0 | 0 |
| CD13 | 99.4 | 99.4 | 99.3 | 99.7 | 99.7 |
| CD29 | 99.6 | 99.2 | 98.7 | 99.5 | 99.5 |
| CD59 | 99.7 | 99.6 | 99.5 | 99.8 | 99.7 |
| CD49e | 98.5 | 95.2 | 98.2 | 95.5 | 97.8 |
| CD151 | 99.2 | 97.7 | 98.4 | 98.7 | 99.2 |
| CD280 | 99 | 96.8 | 98 | 97.4 | 96.6 |
| CD304 | 0 | 0 | 0 | 0 | 0 |
| HLA class I | 99.7 | 98.9 | 99.4 | 99.4 | 99.7 |
| HLA class II | 0.1 | 0.2 | 0.3 | 0 | 0.4 |
| CD274 | 68.5 | 70.4 | 69.9 | 86.2 | 85.2 |
| CD63 | 96 | 89.6 | 93.1 | 96.4 | 96.2 |
| CD106 | 0 | 0 | 0 | 0 | 0 |

### [Example 3] Cell Proliferation Ability

In order to evaluate a cell proliferation ability, an experiment of measuring a doubling time was performed. Here, the doubling time is a time required for cells to be doubled, that is, a time required for one cell to be divided into two cells.

The doubling time of cells was measured by measuring the number of cells for each passage by the following method.
- As described in Example 1 above, 2.5 × 10⁵ P2 cells prepared in Example 1 were seeded in each of three cell culture flasks (#MS-23800 225 cm²: Sumitomo Bakelite Co., Ltd.), and after 5 days (after 2 days from medium replacement), cells were collected and the number of cells was counted (a P3 doubling experiment).
- Similarly, as described in Example 1 above, 2.5 × 10⁵ cells of the P2 cells prepared in Example 1 were seeded in each of three cell culture flasks (#MS-23800 225 cm²: Sumitomo Bakelite Co., Ltd.), and after 5 days (after 2 days from medium replacement), cells were collected and the number of cells was counted (P4 doubling experiment).
- Hereinafter, when subculture was performed by the same method as described in Example 1 and above, the number of cells was counted. When subcultured cells (P5 cells to P10 cells) were obtained, the number of cells was confirmed, and the doubling time was measured.

Results are shown in Table 5. The numbers in Table 5 are doubling times (hr), and "P3" in the upper row indicates the doubling time from P2 to P3 ("P4" indicates the doubling time from P3 to P4: the same for P5 to P10). The doubling time of No.2 to No.5 was 35 hours or less, and in particular, the doubling time of P3, P4, and P5 was 26 hours or less. According to Table 1 in the above Non-Patent Literature 6, the doubling time of cells is generally 25 hours to 53 hours. Therefore, it was confirmed that the above cell populations of No.2 to No.5 proliferated in a very short period of time, with a doubling time of 19 hours to 35 hours.

**[Table 5]**

| Doubling time (h) | | | | | | | | |
|---|---|---|---|---|---|---|---|---|
| | P3 | P4 | P5 | P6 | P7 | P8 | P9 | P10 |
| No2 | 23.46 | 24.63 | 25.49 | 26.58 | 28.30 | 27.68 | 29.50 | 27.82 |
| No3 | 23.05 | 24.49 | 23.77 | 28.68 | 24.71 | 25.31 | 30.10 | 29.65 |
| No4 | 23.08 | 25.00 | 24.09 | 24.54 | 26.26 | 27.04 | 30.38 | 34.56 |
| No5 | 19.62 | 23.52 | 21.45 | 25.22 | 24.47 | 24.53 | 31.23 | 33.08 |

### [Example 4] Cell Viability

In cell transplantation therapy, it is assumed that isolated cells are cryopreserved and thawed for use. Here, it is necessary to maintain a high viability of cryopreserved cells. Therefore, the cell viability was examined using a cell population obtained by culturing mesenchymal cells (four samples) collected from an intraoral tissue.
- About 200 mL of blood was collected from a refractory skin ulcer patient (40 years to 80 years old) caused by venous stasis, and an autologous serum was prepared with CellAid (for serum collection, #JB-SB200CSA: JMS Co., Ltd.).
- Cells were collected from a tissue from a lower part of a salivary gland (an inner cheek) of a refractory skin ulcer patient caused by venous stasis, the tissue was minced with tweezers and a scalpel, and the minced gingival tissue was attached to a 6-well plate (#MS-80060S: Sumitomo Bakelite Co., Ltd.).
- One 50 mL tube was charged with 1.3 mL of autologous serum, 13 mL of AIM-V Medium (Thermo Fisher Scientific K.K.), 13 mL of HFDM-1(+) (Cell Science & Technology Institute, Inc.), 130 µL of Meiji Collagenase G (#COLGS: Meiji Seika Pharma Co., Ltd.), and 52 µL of Meiji Collagenase H (#COLHS: Meiji Seika Pharma Co., Ltd.), and the culture solution was adjusted by pipetting.
- Each well of a 6-well plate was charged with 2 mL of the culture solution, followed by culturing overnight.
- The one 50 mL tube was charged with 1.5 mL of autologous serum, 15 mL of AIM-V Medium (Thermo Fisher Scientific K.K.), and 15 mL of HFDM-1(+) (Cell Science & Technology Institute, Inc.), followed by pipetting to prepare a culture solution.
- The culture solution was removed from the wells of the 6-well plate, and 2 mL of the culture solution was placed in each well of the 6-well plate, and the above-described tissue under a lower part of a salivary gland (an inner cheek) was cultured. P0 cells growing out of the minced tissue were collected and the cells were seeded in a 90 mm Petri dish (#MS-13900S: Sumitomo Bakelite Co., Ltd.). One hour later, the medium was replaced and adhesive cells were cultured. A medium for one 90 mm Petri dish was AIM-V Medium 4.45 mL, HFDM-1(+) 4.45 mL, and NeoSERA 0.5 mL.
- P1 cells were collected when a density of cells cultured in the 90 mm Petri dish reached 70% to 95%, and 2.5 × 10⁵ P1 cells were seeded and cultured in each cell culture flask (#MS-23800S 225 cm²: Sumitomo Bakelite Co., Ltd.). As a culture solution in one cell culture flask, 25 mL of AIM-V Medium + 25 mL of HFDM-1(+) + 1 mL of autologous serum was used.
- The culture solution was removed by suction from the cell culture flask, 25 mL of PBS (Cell Science & Technology Institute, Inc.) was placed in the one cell culture flask, the inside of the cell culture flask was washed, and then PBS was removed by suction.
- The one cell culture flask was charged with 7 mL of a recombinant trypsin/EDTA solution r-TE (Cell Science & Technology Institute, Inc.), and the cell culture flask was allowed to stand in an incubator (37°C, 5% CO₂) for 3 minutes.
- A 40 µm cell strainer (#352340: Corning Incorporated) was attached to a 50 mL tube.
- The cells were detached from the cell culture flask by pipetting and transferred to the 50 mL tube.
- The one cell culture flask was charged with 7 mL of s-TI (Cell Science & Technology Institute, Inc.), which is a synthetic trypsin neutralizer solution, the inside of the 10 cm dish was washed, and the washing solution was transferred to the 50 mL tube.
- The 50 mL tube was centrifuged at 1200 rpm for 2 minutes at 20°C.
- After centrifugation, the supernatant was removed by suction, and the cells were suspended in 10 mL of HFDM-1 (+) (Cell Science & Technology Institute, Inc.) to obtain a suspension containing P2 cells. After 10 µL of the cell suspension liquid and 10 µL of trypan blue were mixed, 10 µL of the mixed liquid was applied to a hemocytometer to count the number of cells.
- A part of P2 cells was adjusted to 1 × 10⁶ cells/mL with STEM-CELLBANKER (registered trademark) GMP grade (product code #CB045: ZENOAQ Resource Co., LTD.), aliquoted into cryotubes at 1 mL per tube to prepare a cell preservation solution containing the P2 cells, which were cryopreserved at -80°C until use.

One of the cryotubes (the cell preservation solution containing P2 cells) which had been cryopreserved at -80°C for a specific time was thawed, and the cell viability was measured by the following method.
- A 15 mL tube was charged with 8.5 mL of AIM-V Medium, 0.5 mL of NeoSERA, and a preservation solution containing the thawed P2 cells, followed by centrifuging (1200 rpm, 2 min, 20°C).
- The supernatant was removed by suction, the cells were suspended in 2 mL of AIM-V medium, the number of cells was counted in 10 µL by the same method as described in Example 1, and the cell viability of P2 was measured.
- Cells were seeded in two 10 cm dishes.
   10 cm dish: AIM-V Medium 5 mL + HFDM-1(+) (Cell Science & Technology Institute, Inc.) + NeoSERA 1 mL, cell suspension liquid 1 mL
- After 4 days to 10 days of culture, the cells were detached from the 10 cm dish using r-TE/s-TI and centrifuged, then the cells were suspended in 2 mL of AIM-V Medium, and the cells were counted at 10 µL to measure the cell viability.

Results are shown in Table 6. As shown in Table 6, the cell viability was maintained high at 71% to 88.5% even after storage at -80°C for 434 to 1457 days.

**[Table 6]**

| | Cell viability [%] | Number of days of cell culture [Day]* | Number of days of storage of cells at -80°C [days]** |
|---|---|---|---|
| No.1 | 88.5 | 22 | 1457 |
| No.2 | 71 | 19 | 1011 |
| No.3 | 84.4 | 18 | 526 |
| No.4 | 78.3 | 29 | 434 |

| | | | |
|---|---|---|---|
| *Number of days in culture from intraoral tissue collection to cell preservation **Number of days since P2 cells are transferred to cryotube and stored at -80°C | | | |

### [Example 5] Preparation of Cell Sheet

Using mesenchymal cells (No.1 to No.5), a cell sheet was produced by the following method.

For a doubling experiment in Example 1, 2.5 × 10⁵ P3 cells were seeded in each of three cell culture flasks (#MS-23800 225 cm²: Sumitomo Bakelite Co., Ltd.), and cells of No.1 were subcultured 3 days after seeding, and cells of No.2 to No.5 were subcultured 5 days after seeding. At this time, the cells were detached from the cell culture flask to count the number of cells, and then P4 cells were seeded in the cell culture flask for the purpose of subculture, and simultaneously 5 × 10⁵ mesenchymal cells were seeded in one well of a 24-well plate and cultured for 72 hours, and the supernatant was stored for ELISA analysis. A cell sheet was prepared from the P4 cells by the method described in the above-described Patent Literature 3. The cell sheet was attached to a CellShifter^{™} so as not to shrink, and then fixed in 10% neutral formalin and subsequently sectioned.

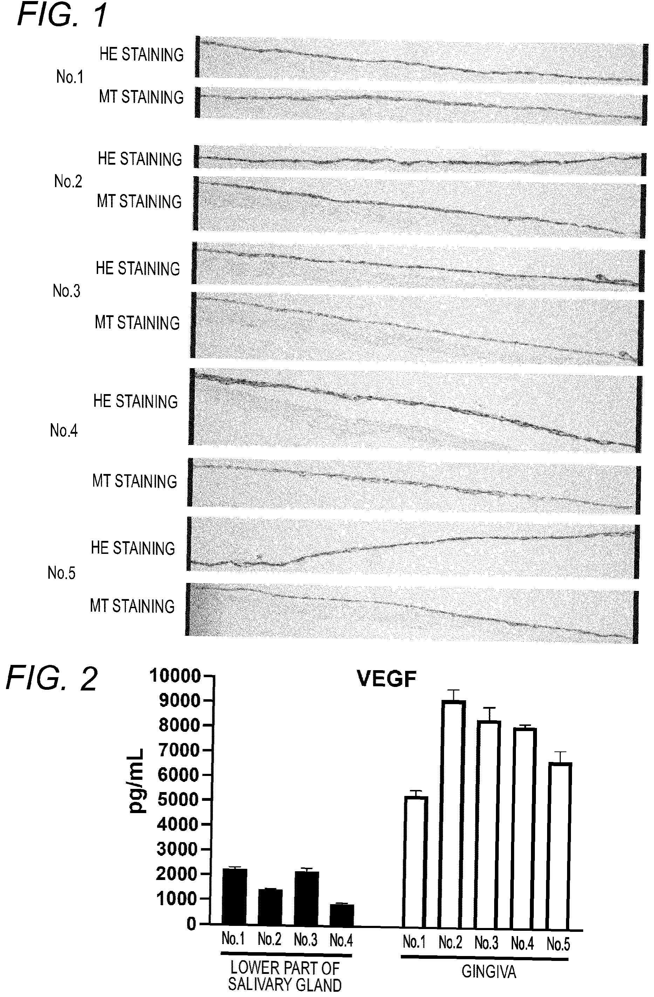

FIG. 1 illustrates a photograph (4x magnification) of a section of the prepared cell sheet stained with hematoxylin-eosin (HE) and MT by a standard method. The cells were confirmed to have a cell sheet structure by the HE staining in FIG. 1. MT staining confirmed that the cell sheet contained an abundant extracellular matrix. It should be noted that cell sheets could be produced in the same manner for P3 cells and P5 to P10 cells (not illustrated).

### [Example 6] Expression of Cell Surface Antigen Molecule of Cells Collected from Intraoral Tissue

In the above Example 2, cells collected from a gingiva were used, but in Example 6, analysis of a cell surface antigen molecule was performed in the same manner as in Example 2 using P4 cells (4 specimens) collected from a tissue from a lower part of a salivary gland (an inner cheek). Cells and a culture method used were the same as in Example 4, and a method for analyzing a cell surface antigen molecule and antibodies and reagents used were the same as in Example 2. Results are shown in Table 7. It was confirmed that the cell population obtained from mesenchymal cells collected from the tissue from a lower part of a salivary gland (an inner cheek) also contained 99% of cells expressing CD90, CD73, CD44, CD13, CD29, or CD59, and contained 27 to 35% of cells expressing CD105, 62% or more of cells expressing CD166, 56% or more of cells expressing CD49e, 93% or more of cells expressing CD151, 97% or more of cells expressing CD280, and 63% or more of cells expressing CD63. On the other hand, cells expressing CD34 and CD45 were not confirmed.

**[Table 7]**

| | No.1 | No.2 | No.3 | No.4 |
|---|---|---|---|---|
| CD90 | 99.2 | 99.7 | 99.6 | 99.4 |
| CD73 | 99.2 | 99.5 | 99.5 | 99.3 |
| CD44 | 99.1 | 99.6 | 99.5 | 99.3 |
| CD105 | 35.4 | 48.1 | 27.8 | 31.9 |
| CD166 | 77.3 | 62.3 | 82.7 | 86.2 |
| CD34 | 0 | 0 | 0 | 0 |
| CD45 | 0 | 0 | 0 | 0 |
| CD13 | 99.2 | 99.6 | 99.6 | 99.3 |
| CD29 | 99.1 | 99.6 | 99.5 | 99.2 |
| CD59 | 99.1 | 99.6 | 99.5 | 99.2 |
| CD49e | 56.6 | 92.3 | 76.8 | 76.5 |
| CD151 | 94.9 | 98.9 | 97 | 93.7 |
| CD280 | 98.6 | 99.3 | 97.3 | 97.4 |
| CD63 | 63.2 | 65.8 | 91.7 | 95.9 |

### [Example 7] Cell Surface Antigen Analysis of Cell Population Obtained by Culturing IFN-γ-treated Mesenchymal Cells Collected from Gingiva

According to the following procedure, cell surface antigen analysis (HLA class I, HLA class II, CD106, CD274) of a cell population obtained by culturing IFN-γ-treated mesenchymal cells was performed.
- One cryotube (the cell preservation solution containing P3) prepared in Example 1 was taken out from -80°C and thawed.
- A 15 mL tube was charged with 3.5 mL of AIM-V Medium, 0.5 mL of NeoSERA, and a thawed cell preservation solution, followed by centrifuging (1200 rpm, 1 min, 20°C).
- The supernatant was removed by suction, and the cells were seeded in two 10 cm dishes and cultured for 3 days (37°C, 5% CO₂). As a medium per 10 cm dish, AIM-V Medium 5 mL + HFDM-1(+) + NeoSERA 1 mL was used.
- The cells were washed with PBS, then detached with r-TE/s-TI, and centrifuged (1200 rpm, 1 min, 20°C). The supernatant was removed by suction, the cells were suspended in 2 mL of AIM-V medium, and the cells were counted at 10 µL.
- Each type of cells was seeded in two 10 cm dishes and cultured overnight. The cells were seeded at 3 × 10⁵ cells per one 10 cm dish.
   One 10 cm dish: AIM-V Medium 5.5 mL + HFDM-1(+) 4.5 mL, NeoSERA 1 mL
- Each of ten 15 mL tubes was charged with 4.5 mL of AIM-V Medium + 4.5 mL of HFDM-1(+) and 1 mL of NeoSERA.
- Five 15 mL tubes were each charged with 5 µL of 0.2 mg/mL (R&D Recombinat Human IFN-r #285-IF/CF, dissolved in Otsuka distilled water (Otsuka Pharmaceutical Factory, Inc.) (final concentration: 100 ng/mL).

The other five 15 mL tubes were each charged with 5 µL of Otsuka distilled water (Otsuka Pharmaceutical Factory, Inc.).
- The culture solution was removed by suction from the 10 cm dish, and the medium prepared as described above was placed thereto, followed by culturing for 48 hours to perform cell surface antigen analysis.

The cell surface antigen analysis was performed in the same manner as in Example 2. The antibodies shown in Table 3 above were used. Results are shown in Tables 8 and 9. CD106 and C274 are shown in separate tables as these were independent experiments.

From Table 8, it was confirmed that the IFN-γ treatment increased the expression of HLA class II. No expression of CD106 was confirmed regardless of the IFN-γ treatment. Further, it was confirmed from Table 9 that the IFN-γ treatment not only increased the expression of HLA class II, but also increased the expression of CD274, resulting in a positive rate of 91% to 96%.

**[Table 8]**

| | No.1 | | No.2 | | No.3 | | No.4 | | No.5 | |
|---|---|---|---|---|---|---|---|---|---|---|
| | control | IFN-γ | control | IFN-γ | control | IFN-γ | control | IFN-γ | control | IFN-γ |
| HLA class I | 99.7 | 99.5 | 99.3 | 99.6 | 99.6 | 99.6 | 99.5 | 99.6 | 99.8 | 99.8 |
| HLA class II | 0.1 | 81.3 | 0.4 | 40.6 | 0.3 | 76.4 | 0.1 | 57.2 | 0.2 | 76.5 |
| CD106 | 0 | 0 | 0 | 0 | 0 | 0 | 0 | 0 | 0 | 0 |

**[Table 9]**

| | No.1 | | No.2 | | No.3 | | No.4 | | No.5 | |
|---|---|---|---|---|---|---|---|---|---|---|
| | control | IFN-γ | control | IFN-γ | control | IFN-γ | control | IFN-γ | control | IFN-γ |
| HLA class I | 99.2 | 99.1 | 99.1 | 99.1 | 99.7 | 99.6 | 99.7 | 99.4 | 99.6 | 99.7 |
| HLA class II | 0.2 | 91.7 | 0.2 | 56.9 | 0.2 | 87.8 | 0.6 | 74.3 | 0.6 | 72.2 |
| CD274 | 65.1 | 95.9 | 70.1 | 94.4 | 62.9 | 95.8 | 57.8 | 96.1 | 68.7 | 91.4 |

MSCs, which are mesenchymal stem cells, have low immunogenicity, but it has been reported that in an inflammatory environment, MSCs acquire immunogenicity through the expression of HLA class II (MHC class II). It has also been reported that cells expressing CD274 (PD-L1) have an action of preventing the function of surrounding immune cells (Yu Yoshinaga et al., Stem Cell Reports. 2022; 17: 1714-1729.).

Therefore, it is considered that in cell transplantation therapy with allogeneic cells, when the transplanted allogeneic cells express CD274, the immunogenicity of the transplanted allogeneic cells is reduced by suppressing attack from immune cells on a living organism side. A ratio of cells expressing CD274 in the cell population established by the present disclosure was 60% or more, but a ratio of cells expressing CD274 in the cell population was 91% or more by culturing in a medium to which IFN-γ was added. This result is considered to be that when the cell population obtained by culturing the mesenchymal cells established by the present disclosure is transplanted as allogeneic cells, strong immunological rejection can be reduced by suppressing attacks from immune cells on the living organism side.

### [Example 8] Secretion of VEGF

For use as a cell sheet, secretion of VEGF is important. The secretion of VEGF from the cell sheet was examined. As a supernatant of a cell sheet prepared from mesenchymal cells collected from a gingival tissue, the supernatant stored for ELISA analysis in the preparation of the cell sheet in Example 5 was used. A supernatant of a cell sheet prepared from mesenchymal cells collected from a tissue from a lower part of a salivary gland (an inner cheek) was prepared by the following method.

The P2 cell preservation solution prepared from the mesenchymal cells collected from the tissue from a lower part of a salivary gland (an inner cheek) which was prepared in Example 4 was taken out from -80°C and thawed. P3 cells were prepared from P2 cells in the same manner as in the method described in Example 1, seeded in a 24-well plate (1.8 cm²/well, MS-80240: Sumitomo Bakelite Co., Ltd.) at 2.78 × 10⁵ cells/cm² (the number of seeds in one well was 5.0 × 10⁵); and cultured in an incubator (37°C, 5% CO₂) for 72 hours. The culture solution was transferred to a 1.5 mL tube, centrifuged at 3000 rpm for 5 minutes at 4°C, and stored at -80°C for ELISA analysis.

In the ELISA analysis, on the day of measurement, a 1.5 mL tube containing the supernatant for each ELISA analysis was thawed on ice, and a VEGF concentration in the supernatant was measured using a Human VEGF Quantikine ELISA Kit (R&D Systems, Inc., #DVE00). For measurement of absorbance and concentration calculation, iMark Microplate Reader (manufactured by Bio-Rad Laboratories, Inc.) and MPM6.exe (manufactured by Bio-Rad Laboratories, Inc.) were used.

### (Results)

FIG. 2 illustrates the VEGF concentration in the medium when a cell sheet was prepared by culturing mesenchymal cells collected from a tissue from a lower part of a salivary gland (an inner cheek) and mesenchymal cells collected from a gingival tissue. From FIG. 2, although all cell sheets were found to secrete sufficient VEGF, the cell sheet prepared from the mesenchymal cells collected from the gingival tissue was confirmed to secrete at least twice as much VEGF as the cell sheet prepared from the mesenchymal cells collected from the tissue from a lower part of a salivary gland (an inner cheek).

### [Example 9] Presence or Absence of Expression of SSEA-3

SSEA-3 is a cell surface antigen molecule known as a representative marker for pluripotent stem cells. Whether gingival-derived mesenchymal cells express SSEA-3 was examined.

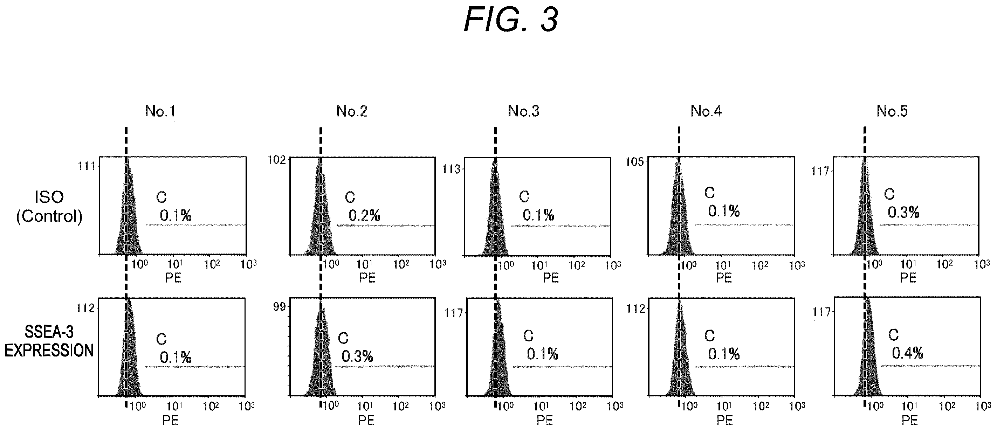

The expression of SSEA-3 was examined in the same manner as in Example 2 using one cryotube (the cell preservation solution containing P3 cells) prepared from gingival-derived mesenchymal cells of five donors No.1 to No.5 by the method described in Example 1. Antibodies used were anti-SSEA-3 antibody (#330311: PE anti-human/mouse SSEA-3 Antibody, BioLegend, Inc.) and a control (#400807: PE Rat IgM, κ Isotype Ctrl Antibody, BioLegend, Inc.). The results are shown in FIG. 3.

As can be seen from FIG. 3, there was no shift of a peak to the right, and positive rate values did not change. Therefore, it was confirmed that the gingival-derived mesenchymal cells did not express SSEA-3.

## Claims

1. A cell population isolated from a living organism, the cell population comprising 80% or more of mesenchymal cells expressing at least one cell surface antigen molecule selected from the group consisting of CD13, CD59, CD49e, CD151, and CD280.

2. The cell population according to claim 1, wherein the mesenchymal cells are cells collected from an intraoral tissue or a culture of the collected cells.

3. The cell population according to claim 1 or 2, wherein the mesenchymal cells express at least three or more cell surface antigen molecules selected from the group consisting of CD13, CD59, CD49e, CD151, and CD280.

4. The cell population according to claim 1 or 2, comprising 60% or more of mesenchymal cells expressing CD274.

5. The cell population according to claim 1 or 2, comprising 5% or less of mesenchymal cells expressing SSEA-3.

6. A three-dimensional cell culture comprising the cell population according to claim 1 or 2.

7. A pharmaceutical composition comprising the cell population according to claim 1 or 2.

8. The pharmaceutical composition according to claim 7, further comprising a pharmaceutically acceptable additive.

9. The pharmaceutical composition according to claim 7 or 8, which is in a form of a three-dimensional cell culture.

10. The pharmaceutical composition according to claim 7 or 8, which is in a form of a cell sheet.

11. An exosome obtained from the cell population according to claim 1 or 2.

12. A method for producing the cell population according to claim 1 or 2, the method sequentially comprising:
a step (a) of collecting mesenchymal cells from a biological tissue; and
a step (b) of culturing the collected mesenchymal cells.

13. The method according to claim 12, further comprising:
a step (c) of using an antibody which recognizes at least one cell surface antigen molecule selected from the group consisting of CD13, CD59, CD49e, CD151, and CD280 to confirm expression of the cell surface antigen molecule, after the step (b).

14. The method according to claim 12 or 13, wherein the step (b) includes culturing in a medium containing an inflammatory cytokine.

15. The method according to claim 12 or 13, further comprising a step (d) of cryopreserving cultured cells, after the step (b).
